# EUROPEAN PATENT SPECIFICATION

(11) **EP 2 142 566 B1**
(45) Date of publication and mention of the grant of the patent: **06.01.2016**
(21) Application number: 08742421.4
(22) Date of filing: 31.03.2008
(51) Int. Cl.: C07K 16/00, A61K 38/00, A61K 39/00, C07K 16/06, C07K 16/12, C07K 14/315

(54) **METHODS AND COMPOSITIONS FOR THE TREATMENT OF PROLIFERATIVE DISEASES**
VERFAHREN UND ZUSAMMENSETZUNGEN ZUR BEHANDLUNG VON PROLIFERATIVEN KRANKHEITEN
PROCEDES ET COMPOSITIONS POUR LE TRAITEMENT DE MALADIES PROLIFERATIVES

(30) Priority: 30.03.2007 US 921169 P
(43) Date of publication of application: 13.01.2010
(73) Proprietor: President and Fellows of Harvard College, Cambridge, MA 02138 (US)
(72) Inventor: QIN, Xuebin, Westwood, MA 02090 (US); HU, Weiguo, Quincy, MA 02170 (US)
(74) Representative: Lahrtz, Fritz
(86) International application number: PCT/US2008/004193
(87) International publication number: WO 2008/121402

(56) References cited:
- WO-A1-2005/108419
- WO-A2-2005/076696
- WO-A2-2008/085366
- NAGAMUNE H ET AL: "A Cell Membrane Modification Technique Using Domain 4 of Intermedilysin for Immunotherapy Against Cancer" ANTICANCER RESEARCH, INTERNATIONAL INSTITUTE OF ANTICANCER RESEARCH, GR, vol. 24, no. 5C, 1 September 2004 (2004-09-01), pages 3367-3372, XP008121527 ISSN: 0250-7005
- OHKURA KAZUTO ET AL: "Structural analysis of human specific cytolysin intermedilysin aiming application to cancer immunotherapy." ANTICANCER RESEARCH 2004 SEP-OCT LNKD- PUBMED:15515430, vol. 24, no. 5C, September 2004 (2004-09), pages 3343-3353, XP08121515 ISSN: 0250-7005
- NAGAMUNE HIDEAKI ET AL: "The human-specific action of intermedilysin, a homolog of streptolysin O, is dictated by domain 4 of the protein." MICROBIOLOGY AND IMMUNOLOGY 2004 LNKD- PUBMED:15383705, vol. 48, no. 9, 2004, pages 677-692, XP008121525 ISSN: 0385-5600
- POLEKHINA GALINA ET AL: "Insights into the action of the superfamily of cholesterol-dependent cytolysins from studies of intermedilysin." PROCEEDINGS OF THE NATIONAL ACADEMY OF SCIENCES OF THE UNITED STATES OF AMERICA 18 JAN 2005 LNKD- PUBMED:15637162, vol. 102, no. 3, 18 January 2005 (2005-01-18), pages 600-605, XP002578303 ISSN: 0027-8424
- HUGHES ET AL: "Characterisation of the high affinity interaction between the human complement regulator CD59 and the Staphylococcus intermedius toxin, intermedilysin" MOLECULAR IMMUNOLOGY, PERGAMON, GB LNKD- DOI:10.1016/J.MOLIMM.2007.06.198, vol. 44, no. 16, 1 September 2007 (2007-09-01), page 3985, XP022227742 ISSN: 0161-5890
- SOLTANI CASIE E ET AL: "Specific protein-membrane contacts are required for prepore and pore assembly by a cholesterol-dependent cytolysin" JOURNAL OF BIOLOGICAL CHEMISTRY, vol. 282, no. 21, May 2007 (2007-05), pages 15709-15716, XP002578304 ISSN: 0021-9258
- SHIMADA Y. ET AL: 'The C-terminal domain of perfringolysin O is an essential cholesterol-binding unit targeting to cholesterol-rich microdomains' EUR. J. BIOCHEM. vol. 269, 2002, pages 6195 - 6203, XP008119981
- WEIS S. ET AL.: 'Streptolysin O: the C terminal tryptophan-rich domain carries functional sites for both membrane binding and self-interaction but not for stable oligomerization' BIOCHIMICA ET BIOPHYSICA ACTA vol. 1510, no. 1-2, 2001, pages 292 - 299, XP004248786
- GIDDINGS K.S. ET AL.: 'Human CD59 is a receptor for the cholesterol-dependent cytolysin intermedilysin' NATURE STRUCTURAL AND MOLECULAR BIOLOGY vol. 11, no. 12, December 2004, pages 1173 - 1178, XP008120005
- GE XIAOWEN ET AL: "rILYd4, a human CD59 inhibitor, enhances complement-dependent cytotoxicity of ofatumumab against rituximab-resistant B-cell lymphoma cells and chronic lymphocytic leukemia.", CLINICAL CANCER RESEARCH : AN OFFICIAL JOURNAL OF THE AMERICAN ASSOCIATION FOR CANCER RESEARCH 1 NOV 2011, vol. 17, no. 21, 1 November 2011 (2011-11-01), pages 6702-6711, ISSN: 1078-0432
- YOU TAO ET AL: "Application of a novel inhibitor of human CD59 for the enhancement of complement-dependent cytolysis on cancer cells.", CELLULAR & MOLECULAR IMMUNOLOGY MAR 2011, vol. 8, no. 2, March 2011 (2011-03), pages 157-163, ISSN: 2042-0226

## Description

### Field of the invention

This invention relates to the treatment of proliferative diseases.

Cancer is a disease marked by the uncontrolled growth of abnormal cells. Cancer cells have overcome the barriers imposed in normal cells, which have a finite lifespan, to grow indefinitely. As the growth of cancer cells continue, genetic alterations may persist until the cancerous cell has manifested itself to pursue a more aggressive growth phenotype. If left untreated, metastasis, the spread of cancer cells to distant areas of the body by way of the lymph system or bloodstream, may ensue, destroying healthy tissue.

CD59 is a glycosylphosphatidylinositol (GPI)-linked membrane protein that is over-expressed in some cancer cells. CD59 activity inhibits formation of the membrane attack complex of complement (MAC) by binding to complement proteins C8 and C9 and preventing C9 incorporation and polymerisation. Complement is a main mediator for antibody mediated cancer cytolysis. Up-regulation and high expression of CD59 is considered to be one of main reasons for resistance to antibody mediated cancer therapy, including resistance to the anti-CD20 chimeric MAb rituximab used for the treatment of B-cell non-Hodgkin lymphoma (B-NHL).

Streptococcus intermedius intermedilysin (ILY) is a cholesterol-dependent cytolysin secreted by Streptococcus intermedius (SI), long suspected to play an important role in the pathogenesis of infectious disease. SI, a gram-positive bacterium, can cause purulent infections in the mouth and internal organs, specifically in the brain and liver. Infections with SI in the brain and liver can lead to abscesses. ILY was assigned to the cholesterol-dependent cytolysin family as the pneumolysin secreted by *Streptococcus pneumoniae* and shows the specific hemolytic activity towards only human erythrocytes, but not towards other animal erythrocytes.

In the prior art, Nagamune et al. (Anticancer Res., 2004, p. 3367-72) disclose a cell membrane modification technique using domain 4 intermedilysin for immunotherapy against cancer.

### Summary of the Invention

In various aspects, the invention uses a substantially pure polypeptide including an ILY domain 4 polypeptide.

In one aspect, the invention provides a pharmaceutical composition including a substantially pure IDLY domain 4 polypeptide and a therapeutic antibody as defined in the claims. In another aspect, the invention provides said polypeptide and/or antibody for use as defined in the claims, e.g. in a method for treating a proliferative disease (e.g., a proliferative disease characterized by neoplastic cells expressing CD59) in patient (e.g., a human) in need thereof by administering to the patient a substantially pure ILY domain 4 polypeptide and a therapeutic antibody. The ILY domain 4 polypeptide and the therapeutic antibody are administered simultaneously, or within 14 days of each other, in amounts that together are sufficient to treat the proliferative disease. In this aspect, the ILY domain 4 polypeptide and therapeutic antibody can be formulated together or separately.

In any of the forgoing aspects, the substantially pure ILY domain 4 polypeptide can include a sequence selected from SEQ ID NO:1 and SEQ ID NO:2, or a fragment thereof. In this aspect, the fragment can be at least 10, 20, 30, 40, 50, 60, 70, 80, 90, 100, or more amino acids in length. The fragment can be fewer than 531, 500, 400, 300, 200, 100, 50, 40, 30, 20, 10, or less amino acids in length.

In any of the forgoing aspects, the therapeutic antibody can be, for example, rituximab, MT201, 17-1A, herceptin, alemtuzumab, lym-1, bevacizumab, cetuximab, or a monoclonal antibody directed to IL-2 receptor alpha.

By "patient" is meant any mammal, e.g., a human, mouse, pig, horse, dog, cat or rat.

By "intermedilysin" or "ILY" is meant a polypeptide having the activity of a *Streptococcus intermedius* intermedilysin polypeptide. ILY can be purified from *Streptococcus intermedius,* or can be produced recombinantly. An exemplary Genbank Accession number corresponding to the nucleic acid sequence of ILY is AB029317 and an exemplary Genbank Accession number corresponding to the polypeptide sequence of ILY is BAE16324. By ILY is also meant a polypeptide with at least 50%, 60%, 70%, 80%, 90%, 95%, or 99% percent sequence identity to the ILY polypeptide. Additionally and alternatively, ILY is defined as a polypeptide encoded by a nucleic acid that hybridizes under high stringency conditions to a nucleic acid of ILY. ILY can be isolated from any *Streptococcus intermedius* strain (e.g., strains 1208-1, UNS35, UNS46, and ATCC27335).

By "domain 4 of ILY polypeptide" or "ILY domain 4 polypeptide" is meant a protein comprising a fragment of ILY having the activity of the ILY domain 4 polypeptide. Specifically excluded from this definition is the full length ILY protein having the Genbank Accession number BAE16324. This term is meant to include a protein containing a peptide sequence GALTLNHDGAFVARFYVYWEELGHDADGYETIRSRSWSGNGYNRGA HYSTTLRFKGNVRNIRVKVLGATGLAWEPWRLIYSKNDLPLVPQRNIS TWGTTLHPQFEDKVVKDNTD (SEQ ID NO:1) or RNIRVKVLGATGLAWEPWRLIYSKNDLPLVPQRNISTWGTTLHPQFED KVVKDNTD (SEQ ID NO:2), or a fragment having ILY domain 4 activity. By ILY domain 4 polypeptide is also meant a polypeptide with at least 50%, 60%, 70%, 80%, 90%, 95%, or 99% percent sequence identity to SEQ ID NO:1 or 2. Additionally and alternatively, ILY domain 4 polypeptide is defined as a polypeptide encoded by a nucleic acid that hybridizes under high stringency conditions to a nucleic acid of the ILY domain 4 polypeptide. The terms are also meant to include any conservative substitutions of amino-acid residues in an ILY domain 4 polypeptide. The term "conservative substitution" refers to replacement of an amino acid residue by a chemically similar residue, e.g., a hydrophobic residue for a separate hydrophobic residue, a charged residue for a separate charged residue, etc. Examples of conserved substitutions for nonpolar R groups are alanine, valine, leucine, isoleucine, proline, methionine, phenylalanine, and tryptophan. Examples of substitutions for polar, but uncharged R groups are glycine, serine, threonine, cysteine, asparagine, or glutamine. Examples of substitutions for negatively charged R groups are aspartic acid or glutamic acid. Examples of substitutions for positively charged R groups are lysine, arginine, or histidine. Furthermore, the term ILY domain 4 polypeptide includes conservative substitutions with non-natural amino-acids. This term explicitly excludes full length ILY.

By "fragment" is meant a portion of a polypeptide that contains, preferably, at least 10%, 20%, 30%, 40%, 50%, 60%, 70%, 80%, 90%, 95%, or more of the entire length of the reference polypeptide. A fragment may contain at least 10, 20, 30, 40, 50, 60, 70, 80, 90, 100, 110, or 114 amino acids or more.

By "ILY domain 4 activity" is meant the activity of a peptide that antagonizes human CD59 but does not directly cause substantial lysis of human red blood cells (RBCs) in the lysis assay described herein.

By "antagonizing human CD59" is meant decreasing the human CD59 binding to complement proteins C8 and C9, resulting in increased formation of the membrane attack complex of complement (MAC).

By "protein" or "polypeptide" or "peptide" means any chain of more than two natural or unnatural amino acids, regardless of post-translational modification (e.g., glycosylation or phosphorylation), constituting all or part of a naturally-occurring or non-naturally occurring polypeptide or peptide, as is described herein.

As used herein, a natural amino acid is a natural α-amino acid having the L-configuration, such as those normally occurring in natural proteins. Unnatural amino acid refers to an amino acid, which normally does not occur in proteins, e.g., an epimer of a natural α-amino acid having the L configuration, that is to say an amino acid having the unnatural D-configuration; or a (D,L)-isomeric mixture thereof; or a homologue of such an amino acid, for example, a β-amino acid, an α,α-disubstituted amino acid, or an α-amino acid wherein the amino acid side chain has been shortened by one or two methylene groups or lengthened to up to 10 carbon atoms, such as an α-amino alkanoic acid with 5 up to and including 10 carbon atoms in a linear chain, an unsubstituted or substituted aromatic (α-aryl or α-aryl lower alkyl), for example, a substituted phenylalanine or phenylglycine.

As used herein, a "peptide for use in the invention" refers to a linear compound comprising the amino acid sequences of an ILY domain 4 polypeptide and containing only natural amino acids which are linked by peptide bonds and which are in an unprotected form.

The present invention also uses derivatives of the peptides for use in the invention. Such derivatives may be linear or circular, and include peptides having unnatural amino acids. Derivatives for use in the invention also include molecules wherein a peptide tor use in the invention is non-covalently or preferably covalently modified by substitution, chemical, enzymatic or other appropriate means with another atom or moiety including another peptide or protein. The moiety may be "foreign" to a peptide for use in the invention as defined above in that it is an unnatural amino acid, or in that one or more natural amino acids are replaced with another natural or unnatural amino acid. Conjugates comprising a peptide or derivative for use in the invention covalently attached to another peptide or protein are also encompassed herein. Attachment of another moiety may involve a linker or spacer, e.g., an amino acid or peptidic linker. Derivatives for use in the invention also included peptides wherein one, some, or all potentially reactive groups, e.g., amino, carboxy, sulfhydryl, or hydroxyl groups are in a protected form.

The atom or moiety derivatizing a peptide for use in the invention may serve analytical purposes, e.g., facilitate detection of the peptide for use in the invention, favor preparation or purification of the peptide, or improve a property of the peptide that is relevant for the purposes of the present invention. Such properties include binding to an human CD59 or suitability for *in vivo* administration, particularly solubility or stability against enzymatic degradation. Derivatives for use in the invention include a covalent or aggregative conjugate of a peptide for use in the invention with another chemical moiety, the derivative displaying essentially the same activity as the underivatized peptide for use in the invention, and a "peptidomimetic small molecule" which is modeled to resemble the three-dimensional structure of any of the amino acids in context with the invention. Examples of such mimetics are retro-inverso peptides (Chorev et al., Acc. Chem. Res. 26: 266-273, 1993). The designing of mimetics to a known pharmaceutically active compound is a known approach to the design of drugs based on a "lead" compound. This may be desirable, e.g., where the "original" active compound is difficult or expensive to synthesize, or where it is unsuitable for a particular mode of administration, e.g., peptides are considered unsuitable active agents for oral compositions as they tend to be quickly degraded by proteases in the alimentary canal.

Additional examples of derivatives within the above general definitions include the following:
(I) Cyclic peptides or derivatives including compounds with a disulfide bridge, a thioether bridge, or a lactam. Typically, cyclic derivatives containing a disulphide bond will contain two cysteines, which may be L-cysteine or D-cysteine. Advantageously, the N-terminal amino acid and the C-terminal amino acids are both cysteines. In such derivatives, as an alternative to cysteine, penicillamine (β,β-dimethyl-cysteine) can be used. Peptides containing thioether bridges are obtainable, e.g., from starting compounds having a free cysteine residue at one end and a bromo-containing building block at the other end (e.g., bromo-acetic acid). Cyclization can be carried out on solid phase by a selective deprotection of the side chain of cysteine. A cyclic lactam may be formed, e.g., between the γ-carboxy group of glutamic acid and the ε-amino group of lysine. As an alternative to glutamic acid, it is possible to use aspartic acid. As an alternative to lysine, ornithine or diaminobutyric acid may be employed. Also, it is possible to make a lactam between the side chain of aspartic acid or glutamic acid at the C-terminus and the α-amino group of the N-terminal amino acid. This approach is extendable to β-amino acids (e.g., β-alanine). Alternatively, glutamine residues at the N-terminus or C-terminus can be tethered with an alkenedyl chain between the side chain nitrogen atoms (Phelan et al., J. Amer. Chem. Soc. 119:455-460, 1997).
(II) Peptides for use in the invention, which are modified by substitution. In one example, one or more, preferably one or two, amino acids are replaced with another natural or unnatural amino acid, e.g., with the respective D-analog, or a mimetic. For example, in a peptide containing Phe or Tyr, Phe or Tyr may be replaced with another building block, e.g., another proteinogenic amino acid, or a structurally related analogue. Particular modifications are such that the conformation in the peptide is maintained. For example, an amino acid may be replaced by a α,α-disubstituted amino acid residue (e.g., α-aminoisobutyric acid, 1-amino-cyclopropane-1-carboxylic acid, 1-amino-cycloperitane-1-carboxylic acid, 1 -amino-cyclohexane-1 -carboxylic acid, 4-amino piperidine-4-carboxylic acid, and 1-amino-cycloheptane-1-carboxylic acid).
(III) Peptides for use in the invention detectably labeled with an enzyme, a fluorescent marker, a chemiluminescent marker, a metal chelate, paramagnetic particles, biotin, or the like. In such derivatives, the peptide for use in the invention is bound to the conjugation partner directly or by way of a spacer or linker group, e.g., a (peptidic) hydrophilic spacer. Advantageously, the peptide is attached at the N- or C-terminal amino acid. For example, biotin may be attached to the N-terminus of a peptide for use in the invention via a serine residue or the tetramer Ser-Gly-Ser-Gly.
(IV) Peptides for use in the invention carrying one or more protecting groups at a potentially reactive side group, such as amino-protecting group, e.g., acetyl, or a carboxy-protecting group. For example, the C-terminal carboxy group of a compound for use in the invention may be present in form of a carboxamide function. Suitable protecting groups are commonly known in the art. Such groups may be introduced, for example, to enhance the stability of the compound against proteolytic degradation.

By a "derivative" of a peptide for use in the invention is also meant a compound that contains modifications of the peptides or additional chemical moieties not normally a part of the peptide. Modifications may be introduced into the molecule by reacting targeted amino acid residues of the peptide with an organic derivatizing agent that is capable of reacting with selected side chains or terminal residues. Methods of derivatizing are described below.

Cysteinyl residues most commonly are reacted with α-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, α-bromo-β-(5-imidozoyl) propionic acid, chloroacetyl phosphate, N-alkylmaleimides, 3-nitro-2-pyridyl disulfide, methyl 2-pyridyl disulfide, p-chloromercuribenzoate, 2-chloromercuri-4-nitrophenol, or chloro-7-nitrobenzo-2-oxa-1,3-diazole.

Histidyl residues are generally derivatized by reaction with diethylprocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing α-amino-containing residues include imidoesters such as methyl picolinimidate; pyridoxal phosphate; pyridoxal; chloroborohydride; trinitrobenzenesulfonic acid; O-methylissurea; 2,4-pentanedione; and transaminase-catalyzed reaction with glyoxylate.

Arginyl residues are modified by reaction with one or several conventional reagents, among them phenylglyoxal, 2,3-butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

Carboxyl side groups (aspartyl or glutamyl) are selectively modified by reaction with carbodiimides (R'--N--C--N--R') such as 1-cyclohexyl-3-(2-morpholinyl-(4-ethyl) carbodiimide or 1-ethyl-3 (4 azonia 4,4-dimethylpentyl) carbodiimide. Aspartyl and glutamyl residues can also be converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

Polypeptides or derivatives thereof may be fused or attached to another protein or peptide, e.g., as a glutathione-S-transferase (GST) fusion polypeptide. Other commonly employed fusion polypeptides include, but are not limited to, maltose-binding protein, *Staphylococcus aureus* protein A, polyhistidine, and cellulose-binding protein.

By a "peptidomimetic small molecule" of a peptide is meant a small molecule that exhibits substantially the same ILY domain 4 activity as the peptide itself.

By "substantially pure polypeptide" is meant a polypeptide or peptide that has been separated from the components that naturally accompany it. Typically, the polypeptide is substantially pure when it is at least 60%, by weight, free from the proteins and naturally-occurring organic molecules with which it is naturally associated. Preferably the polypeptide is an ILY domain 4 polypeptide that is at least 75%, more preferably at least 90%, and most preferably at least 99%, by weight, pure. A substantially pure ILY domain 4 polypeptide may be obtained, for example, by extraction from a natural source (e.g., a fibroblast, neuronal cell, or lymphocyte) by expression of a recombinant nucleic acid encoding an ILY domain 4 polypeptide, or by chemically synthesizing the polypeptide. Purity can be measured by any appropriate method, e.g., by column chromatography, polyacrylamide gel electrophoresis, or HPLC analysis.

A protein is substantially free of naturally associated components when it is separated from those contaminants that accompany it in its natural state. Thus, a protein that is chemically synthesized or produced in a cellular system different from the cell from which it naturally originates will be substantially free from its naturally associated components. Accordingly, substantially pure polypeptides include those derived from eukaryotic organisms but synthesized in *E. coli* or other prokaryotes.

The "percent sequence identity" of two nucleic acid or polypeptide sequences can be readily calculated by known methods, including but not limited to those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, Academic Press, 1987; and Sequence Analysis Primer, Gribskov, and Devereux, eds., M. Stockton Press, New York, 1991; and Carillo and Lipman, SIAM J. Applied Math. 48:1073, 1988.

Methods to determine identity are available in publicly available computer programs. Computer program methods to determine identity between two sequences include, but are not limited to, the GCG program package (Devereux et al., Nucleic Acids Research 12:387, 1984), BLASTP, BLASTN, and FASTA (Altschul et al., J. Mol. Biol. 215:403, 1990). The well known Smith Waterman algorithm may also be used to determine identity. The BLAST program is publicly available from NCBI and other sources (BLAST Manual, Altschul, et al., NCBI NLM NIH Bethesda, Md. 20894). Searches can be performed in URLs such as the following: http://www.ncbi.nlm.nih.govBLAST/unfinishedgenome.html; or http://www.tigr.org/cgi-binBlastSearch/blast.cgi. These software programs match similar sequences by assigning degrees of homology to various substitutions, deletions, and other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine.

By "hybridize" is meant to form a double-stranded complex containing complementary paired nucleobase sequences, or portions thereof, under various conditions of stringency. (See, e.g., Wahl. and Berger, Methods Enzymol. 152:399 (1987); Kimmel, Methods Enzymol. 152:507 (1987))

By "hybridizes under high stringency conditions" is meant under conditions of stringent salt concentration, stringent temperature, or in the presence of formamide. For example, stringent salt concentration will ordinarily be less than about 750 mM NaCl and 75 mM trisodium citrate, preferably less than about 500 mM NaCl and 50 mM trisodium citrate, and most preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and most preferably at least about 50% formamide. Stringent temperature conditions will ordinarily include temperatures of at least about 30° C, more preferably of at least about 37° C, and most preferably of at least about 42° C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred embodiment, hybridization will occur at 30° C in 750 mM NaCl, 75 mM trisodium citrate, and 1% SDS. In a more preferred embodiment, hybridization will occur at 37° C in 500 mM NaCl, 50 mM trisodium citrate, 1% SDS, 35% formamide, and 100 µg/ml denatured salmon sperm DNA (ssDNA). In a most preferred embodiment, hybridization will occur at 42° C in 250 mM NaCl, 25 mM trisodium citrate, 1% SDS, 50% formamide, and 200 µ g/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25° C, more preferably of at least about 42° C, and most preferably of at least about 68° C. In a preferred embodiment, wash steps will occur at 25° C in 30 mM NaCl, 3 mM trisodium citrate, and 0.1% SDS. In a more preferred embodiment, wash steps will occur at 42° C in 15 mM NaCl, 1.5 mM trisodium citrate, and 0.1% SDS. In a most preferred embodiment, wash steps will occur at 68° C in 15 mM NaCl, 1.5 mM trisodium citrate, and 0.1 % SDS. Additional variations on these conditions will be readily apparent to those skilled in the art. Hybridization techniques are well known to those skilled in the art and are described, for example, in Benton and Davis (Science 196:180 (1977)); Grunstein and Hogness (Proc. Natl. Acad. Sci. USA 72:3961 (1975)); Ausubel et al. (Current Protocols in Molecular Biology, Wiley Interscience, New York (2001)); Berger and Kimmel (Guide to Molecular Cloning Techniques, Academic Press, New York, (1987)); and Sambrook et al. (Molecular Cloning: A Laboratory Manual, Cold Spring Harbor Laboratory Press, New York). Preferably, hybridization occurs under physiological conditions. Typically, complementary nucleobases hybridize via hydrogen bonding, which may be Watson-Crick, Hoogsteen or reversed Hoogsteen hydrogen bonding, between complementary nucleobases. For example, adenine and thymine are complementary nucleobases that pair through the formation of hydrogen bonds.

By "therapeutic antibody" is meant a pharmaceutical composition containing an antibody or antibody derivative formulated to treat a proliferative disease.

### Brief Description of the Drawings

Figure 1A is a graph showing percent lysis of red blood cells (RBCs) when treated with ILY. The RBCs of *hCD59RBC^{+l-}* mice, but not those of wild type (WT) mice are hyper-sensitive to *ex vivo* ILY mediated lysis, at a level comparable to that of human RBC.
Figure 1B is a graph showing the induction of hemolysis in *hCD59RBC*^{+/-} resulting from an ILY (45 ng ILY/g body weight) tail vein injection. The blood was collected from the mouse tail vein and hematocrit values were measured as described previously.
Figure 1C is a photograph showing visible hemolysis in samples collected from five *hCD59RBC*^{+/-} mice, but not in those samples from five WT mice. Samples were processed in hematocrit tubes obtained from mouse vein tail 10 minutes after ILY injection (45 ng/g body weight).
Figure ID is a photograph showing hemoglobinuria in *hCD59RBC*^{+/-} mice but not in WT mice at 5 hours and 1 day after ILY administration (45 ng ILY/g body weight). The urine was collected in metabolic cages as described previously.
Figure 2A is a graph showing percent ILY mediated hemolysis. To evaluate the protective effect of human serum against ILY-mediated hemolysis of serum, serial dilutions of ILY were added to human RBC incubated with a 1 to 8 dilution of serum from different species as described previously.
Figure 2B is a graph showing percent ILY mediated hemolysis as a functional comparison of the eluted fraction with the flow through from the ILY column. The eluted fraction (triangles) and the flow through (rectangles) from ILY column were dialyzed and concentrated to volume equal to amount of the human serum from (crosses) that was loaded on the ILY column. ILY concentration for hemolytic assay is 1.6x 10⁻⁹ M. This data represents an experiment repeated three times.
Figure 2C is a western blot showing the isolation of ILY-binding proteins from human serum. The lanes were loaded as follows: 1: 0.8 µl of human serum, 2: 0.8 µl flow through from human serum loaded on the ILY-binding column, 3: 10 µg proteins from the eluted fraction of human serum loaded on ILY column, 4: 0.8 µl of mouse serum, 5: 0.8 µl flow through from mouse serum loaded on ILY-binding column, 6: 10 µg protein from the eluted fraction of mouse serum loaded on ILY column. Arrows indicate bands cut for protein sequencing.
Figure 2D is a table showing protein sequencing information.
Figure 3A is a western blot showing further isolation of ILY-binding human IgG with protein G column. Lanes were loaded as follows: 1: protein maker; 2: 10 µl of the fraction eluted from ILY column; 3: 20 µl of the eluted fraction after further purification with protein G column; 4: 20 µl of the flow through from further purification with protein G column. Samples used for lanes 2, 3, and 4 were equalized to the same volume by dialysis and concentration. Total original volume of the each sample of lane 2, lane 3, and lane 4 are equal.
Figure 3B is a graph showing percent ILY mediated hemolysis with the inhibitory effect of human ILY-binding IgG. The volumes of the eluted fraction from ILY column (crosses), the eluate from further purification with protein G column (rectangles), and the flow through from further purification with protein G column (triangles) were equalized by dialysis and concentration. ILY concentration for the hemolytic assay is 1.6x 10⁻⁹ M. This data represents an experiment repeated three times.
Figure 3C is a graph showing percent ILY mediated hemolysis in the absence of an inhibitory effect of mouse ILY-binding IgG. The volumes of mouse and human serum loaded on the ILY-binding column were equal. The total volume of the eluted fraction from mouse serum (rectangles), flow through from mouse serum (crosses), and the eluted fraction from human serum (triangles) were equalized by dialysis and concentration. ILY concentration is 1.6x 10⁻⁹ M.
Figure 3D is a graph showing ILY binding to the Fab region of human ILY-binding. 200 ng ILY was used to coat each well.
Figure 4A is a diagram showing different ILY fragments. The domains present in each fragment (domains 1-4) are indicated. ILY domains 1, 2, 3 and 4 are represented by squares with different shading.
Figure 4B is an SDS-PAGE gel stained with Coomassie Blue showing recombinant ILY purified from a bacterial expression system using a HIS column.
Figure 5A is a graph showing percent lysis of cells treated with the indicated ILY fragment.
Figure 5B is a graph showing percent lysis as a function of the indicated ILY fragment concentration. Human erythrocytes were preincubated with mILY3 and mILY4 for 30 min at room temperature prior to exposure to ILY (1.2 X 10⁻⁹ M). In cells not preincubated with an ILY fragment, this concentration is sufficient to induce 100% cell lysis.
Figure 6 is a graph showing percent hemolysis as a function of mILY3 concentration in hCD59RBC transgenic mouse erythrocytes. Antibody sensitized ThCD59RBC's and wild type RBCs were pre-incubated with different concentrations of mILY3 and exposed to human complement. The increased sensitivities to human complement mediated hemolysis in hCD59RBC erythrocytes correlates with the concentration of mILY3.
Figure 7 is a graph showing fluorescent intensity as a function of cell number. The shaded curve represents RAMOS cells stained with secondary FITC antibody alone. The white curve represents cells stained with anti-human CD59 antibody and FITC conjugated secondary antibody. Cells were treated with the indicated concentration of rituximab and 10% serum.
Figure 8A is a graph showing percent cell death as a function of mILY3 peptide concentration in RAMOS cells preincubated with the indicated concentration of mILY3 and 51.2 µg of rituximab.
Figure 8B is a series of photomicrographs showing cells stained with TrypanBlue in samples containing RAMOS cells treated with the indicated concentrations of mILY3, 10 µg rituximab, and 10% of heat inactivated fetal bovine serum.

### Detailed Description

In general, the invention features pharmaceutical formulations comprising peptide fragments containing domain 4 of the *Streptococcus intermedius* intermedilysin (ILY) protein and the use of these fragments to sensitize cancer cells to antibody-based anticancer treatments. CD59 receptor activity has been associated with decreased sensitivity to therapeutic antibodies. Administration of ILY domain 4 polypeptides is sufficient to inhibit CD59 receptor activity while avoiding the general toxicity associated with full length ILY.

### I. Methods of Administration

Therapy in context with the invention may be performed alone or in conjunction with another therapy and may be provided at home, the doctor's office, a clinic, a hospital's outpatient department, or a hospital. Treatment optionally begins at a hospital so that the doctor can observe the therapy's effects closely and make any adjustments that are needed, or it may begin on an outpatient basis. The duration of the therapy depends on the type of disease or disorder being treated, the age and condition of the patient, the stage and type of the patient's disease, and how the patient responds to the treatment. Additionally, a person having a greater risk of developing an proliferative disease may receive treatment to inhibit or delay the onset of symptoms.

Routes of administration for the various embodiments include, but are not limited to, topical, transdermal, transcranial, nasal, and systemic administration (such as, intravenous, intramuscular, subcutaneous, inhalation, rectal, buccal, vaginal, intraperitoneal, intraarticular, ophthalmic, otic, or oral administration). As used herein, "systemic administration" refers to all nondermal routes of administration, and specifically excludes topical and transdermal routes of administration.

### Dosages

The dosage of peptides for use in the invention depends on several factors, including: the administration method, the disease to be treated, the severity of the disease, whether the disease is to be treated or prevented, and the age, weight, and health of the person to be treated. Additionally, pharmacogenomic (the effect of genotype on the pharmacokinetic, pharmacodynamic or efficacy profile of a therapeutic) information about a particular patient may affect dosage used.

Continuous daily dosing with the peptides for use in the invention may not be required. A therapeutic regimen may require cycles, during which time a drug is not administered, or therapy may be provided on an as needed basis during periods of acute inflammation.

As described above, the peptides for use in the invention may be administered orally in the form of tablets, capsules, elixirs or syrups, or rectally in the form of suppositories. The peptides may also be administered topically in the form of foams, lotions, drops, creams, ointments, emollients, or gels. Parenteral administration of a compound is suitably performed, for example, in the form of saline solutions or with the compound incorporated into liposomes

### II. Indications

The compositions and medical uses of the invention are useful for treating any disease characterized by undesired human CD59 activity, including those set forth below.

The compositions and medical uses of the invention are useful for the treatment of cancers and other disorders characterized by hyperproliferative cells (proliferative diseases). In these embodiments, an ILY domain 4 polypeptide can be administered directly to a CD59-expressing neoplasia, or systemically to a subject having a neoplasia. Preferably, the ILY domain 4 polypeptide will be administered with an anti-cancer therapeutic antibody.

In a separate embodiment, the ILY domain 4 polypeptide can be administered to a patient diagnosed with a proliferative disorder that is not characterized by the cell surface expression of CD59. Here, the ILY domain 4 polypeptide is administered in conjunction with an anti-cancer therapeutic antibody to prevent resistance to the therapeutic antibody based treatment.

Therapy may be performed alone or in conjunction with another therapy (e.g., surgery, radiation therapy, chemotherapy, immunotherapy, anti-angiogenesis therapy, or gene therapy). The duration of the therapy depends on the type of disease or disorder being treated, the age and condition of the patient, the stage and type of the patient's disease, and how the patient responds to the treatment. Therapy may be given in on-and-off cycles that include rest periods so that the patient's body has a chance to recovery from any as yet unforeseen side-effects. Desirably, the medical uses and compositions of the invention are more effective than other methods and compositions. By "more effective" is meant that a method, composition, or kit exhibits greater efficacy, is less toxic, safer, more convenient, better tolerated, or less expensive, or provides more treatment satisfaction than another method, composition, or kit with which it is being compared.

Cancers include, without limitation, leukemias (e.g., acute leukemia, acute lymphocytic leukemia, acute myelocytic leukemia, acute myeloblastic leukemia, acute promyelocytic leukemia, acute myelomonocytic leukemia, acute monocytic leukemia, acute erythroleukemia, chronic leukemia, chronic myelocytic leukemia, chronic lymphocytic leukemia), polycythemia vera, lymphoma (Hodgkin's disease, non-Hodgkin's disease), Waldenstrom's macroglobulinemia, heavy chain disease, and solid tumors such as sarcomas and carcinomas (e.g., fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, uterine cancer, testicular cancer, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodenroglioma, schwannoma, meningioma, melanoma, neuroblastoma, and retinoblastoma).

### III. Anti-cancer therapeutic antibodies

The invention features medical uses for the treatment of proliferative diseases through the administration of an ILY domain 4 polypeptide in combination with a therapeutic antibody. Administration of the ILY domain 4 polypeptide sensitizes the cells targeted by antibody therapy to complement-mediated cell lysis. Examples of therapeutic antibodies for use in the invention are set forth in Table 1.

**Table 1: Relevant antibodies that are applied for cancer therapy**

| Antibody name | Target antigen | Cancer |
|---|---|---|
| Rituximab | CD20 | B-cell lymphomas |
| MT201 and 17-1A | Ep-CAM | Colorectal and breast cancer |
| Herceptin | HER2 | Breast cancer and lymphomas |
| Alemtuzumab | CD52 | Chronic lymphocytic leukemia |
| Lym-1 | HLA-DR | Lymphoma |
| Bevacizumab | VEGF | Cancer of the colon or rectum |
| Cetuximab | EGFR | colorectal cancer |
| IL-2Rα-directed monoclonal antibodies | | T-cell leukemia |

In addition to the antibodies and indications listed above, the invention considers co-administration of the ILY domain 4 polypeptide in combination with any treatment that would be enhanced by inhibition of human CD59 activity.

### IV. Experimental Results

*Streptococcus intermedius* (SI) is part of the normal human oral micro flora and can cause liver and brain abscesses. ILY secreted by SI specifically binds and lyses CD59 positive human cells. Our results demonstrate that human serum, but not the serum from any other tested species, can neutralize the lytic function of ILY. We have identified an ILY-binding human immunoglobulin (IgG) purified from human serum that exhibits a functional inhibitory effect on ILY-mediated hemolysis *ex vivo* and *in vivo.*

Using FACS analysis with anti-CD59 antibodies and RT-PCR, CD59 expression has been detected on the surface of adipocytes at both the protein and mRNA level. CD59 is also highly expressed in sperm cells and has been suspected to play an important role in male fertility. Deficiencies in mouse CD59 expression results in the progressive loss of male fertility, suggesting a role of CD59 in male reproduction.

CD59 is also highly expressed in various cancer cells such as prostate, breast and gastric adenomas and intestinal-type gastric carcinomas, and B-cell lymphoma. Higher expression levels of CD59 in neoplastic cells have been correlated with cellular resistance to certain chemotherapeutic drugs. We have demonstrated that ILY specifically binds to human CD59 and lyses human cells that express human CD59 on the cell surface. We found that humans develop specific immunity to protect cells from ILY-mediated cell lysis.

### 1. Experimental model

We used a plasmid containing His-tagged ILY to express and purify recombinant ILY from bacteria using a His Bind Purification Kit (Novagen). In order to show that hCD59 is the only receptor for ILY in human RBC, we have performed *ex vivo* and *in vivo* experiments with *hCD59RBC.*

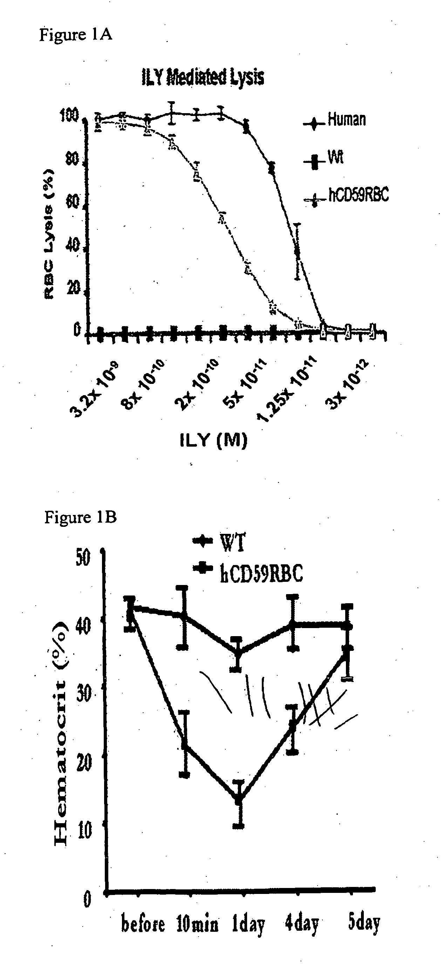

For the *ex vivo* study, we demonstrated that the expression of hCD59 in the RBC of *hCD59RBC* transgenic mice makes the *hCD59RBC*^{+/-} mRBCs hyper-sensitive to ILY-mediated lysis. This lysis is comparable to the level of ILY-mediated lysis of human RBC (Figure 1A). WT mRBC are resistant to ILY-mediated lysis. For the *in vivo* study, we administrated different doses of ILY by tail vein injection. With three different doses of ILY (95, 47, 30 ng/g body weight), the percentages of ILY-induced cell death of *hCD59RBC* transgenic mice were 100% (15/15 animals), 50% (8/16 animals) and 0% (0/6 animals), respectively. Based on this data, we consider the dose of 95 ng ILY/g body weight as the lethal dose (LD₁₀₀) in our *in vivo* experiments.

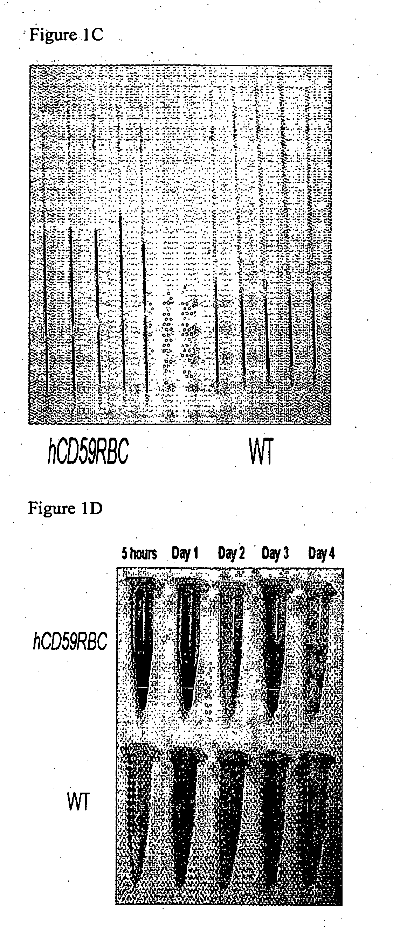

There was no observed cell death in RBCs from WT mice (0/6 animals), even at the highest levels of treatment (3000ng ILY/g body weight of ILY). Injecting 45 ng ILY/g body weight induced massive hemolysis in *hCD59RBC*^{+/-} but not in WT mice, as demonstrated by decreased the hematocrit values and visible hemolysis and hemoglobinuria. Figure 1B shows significantly reduced hematocrit values in *hCD59RBC*^{+/-} as compared to WT mice at 10 minutes, 1 day, and 4 day after ILY injection. Visible hemolysis was found in five *hCD59RBC*^{+/-} mice, but not in five WT mice at 10 minutes after ILY injection (Figure 1C). Visible hemogloabinuria was found in the urine from *hCD59RBC*^{+/-} but not in WT mice, when collected at 5 hours and 1 day after ILY injection (Figure 1D).

### 2. Inhibitors in human serum neutralize the lytic effect of ILY

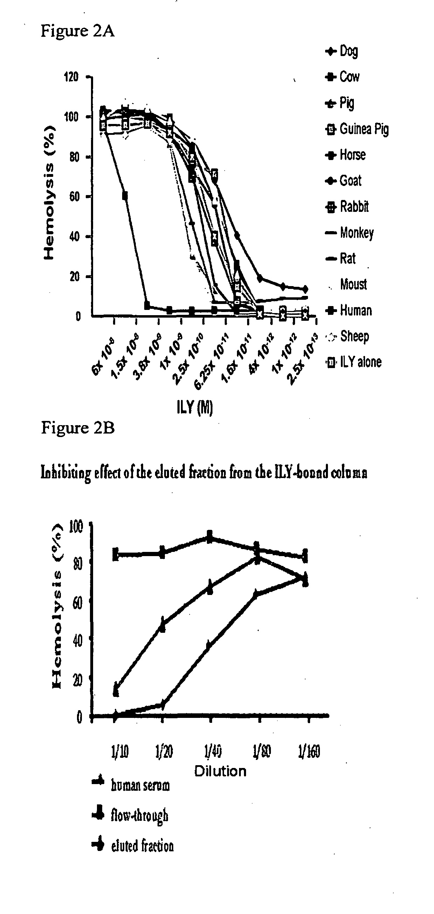

Because SI (1) is part of the normal human oral micro flora, (2) can cause liver and brain abscesses, and (3) secretes ILY that specifically lyses human cells due to the presence of hCD59 in their surface, we propose that humans may develop some immune defenses that protect against ILY-mediated cell lysis and pathogenic SI infection. In order to test this hypothesis, we performed hemolytic assays with human RBC. Human RBC were incubated with different concentrations of ILY and a 1 to 8 dilution of serum in PBS from different species. Figure 2A shows that human serum, and not the serum from other 11 animal species, significantly blocked ILY-mediated human RBC lysis.

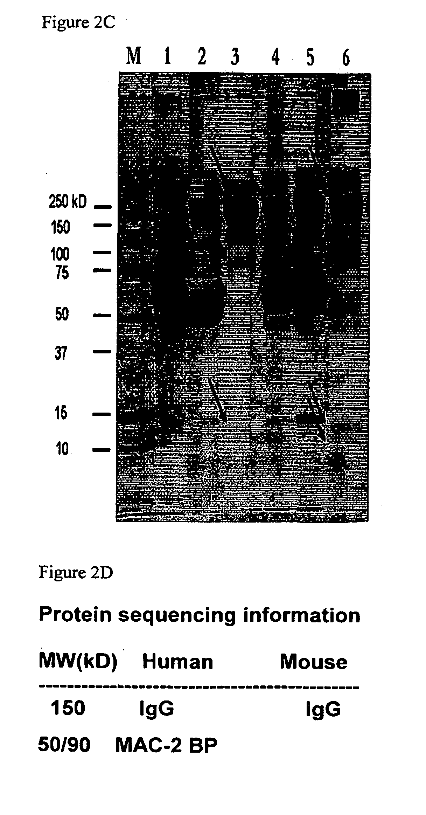

In order to isolate the inhibitors from human serum, we used an ILY-binding sepharose column. By hemolytic assay, we verified that the eluted fraction and not the flow through of human serum has functional activity that protects human RBC from ILY-mediated hemolysis (Figure 2B). The specific proteins that bound to the ILY were separated on SDS-PAGE gel (Figure. 2C) and were isolated for protein sequencing. We found IgG in the ILY-eluted fractions of human and mouse serum. MAC-2BP protein was only found in the ILY-eluted fraction of human serum (Figure. 2D).

### 3. Isolation of ILY inhibitors and their protective effect

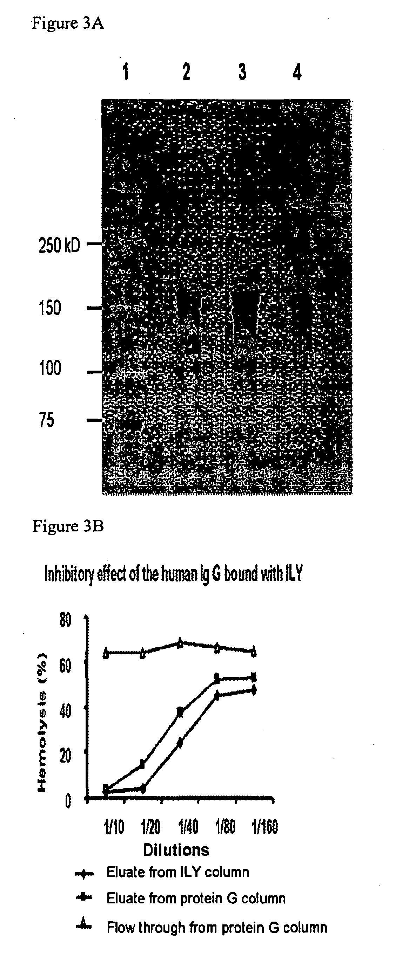

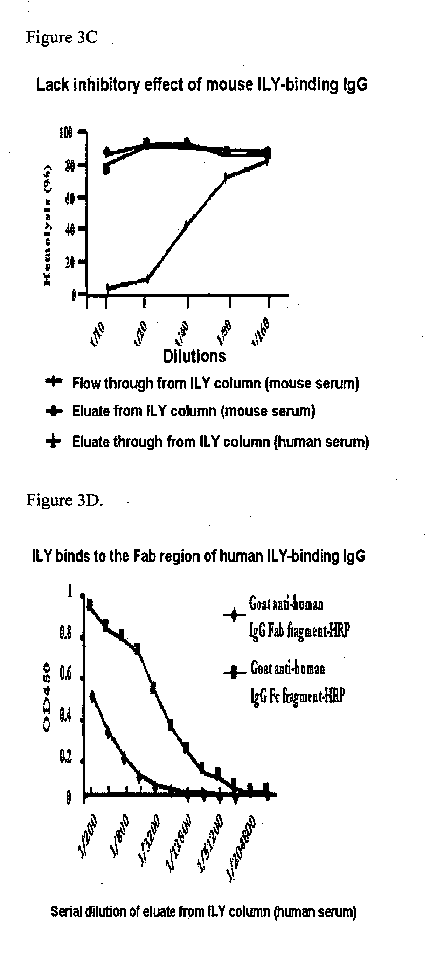

In order to separate the human IgG or mouse IgG from MAC-2BP and SP40, we further purified the eluted fraction from the ILY-binding column with a protein G column, which binds to IgG (Figure 3B). We used a hemolytic assay to test the functional activity of the eluted fractions from human serum purified on the ILY column and the protein G column. Figure 3B shows the eluted fraction from the protein G column has a functional activity similar to that of the eluted fraction from the ILY column. We have purified approximately 300 µg of ILY-binding human IgG or mouse IgG from 1 ml of each species' serum. Normally, there is an average of 12 mg/ml of total human IgG in human serum. Therefore, 2.5% of the total human IgG can bind to ILY. Only the ILY-binding IgG from human serum, and not from mouse serum, has functional activity in protecting hRBC from ILY-mediated hemolysis (Figure. 3B). ILY-binding mouse IgG does not have any protective activity, against ILY-mediated lysis (Figure. 3C).

A possible explanation for why the 2.5% of mouse IgG that specifically binds to ILY does not block ILY mediated cell lysis follows. First, SI is part of the normal micro flora only in humans, not in the mouse and other animals. Thus, the human immune system can be exposed to ILY and produce antibodies specific to the functional domain of ILY, including the domain 4 (a binding site for hCD59). Second, there may be cross-reactivity of ILY with antibodies against the cytolytic toxins that are produced by other Gram-positive bacteria, including species within the genera *Clostridium*, S*treptococcus*, *Listeria*, and *Bacillus*, which are normal microflora in humans and other animals. The members of this toxin family-including ILY-exhibit 40-80% similarity at the primary sequence level. Humans and other animals can produce antibodies specific for a variety of these toxins. It is possible that these antibodies can cross react with different regions of ILY, but not neutralize ILY function.

In order to further determine which part of this ILY-binding human IgG molecule binds to ILY, 200 ng ILY was used to coat wells in a 96-well plate followed by incubation with serial dilutions of the eluate from human serum loaded on the ILY-binding column. Saturating amounts of secondary antibodies (goat anti-human IgG Fab or Fc fragment-HRP antibodies) were used to detect free binding sites in the wells after the ILY-coated wells were pre-incubated with serial dilutions of human serum eluate. Figure 3D shows significantly more-free binding site for anti-human Fc secondary antibody than for anti-human Fab secondary antibody. This result suggests that the site where the ILY-binding human IgG binds to ILY may reside in the Fab region. Furthermore, using ELISA, we demonstrated that ILY does not bind to the Fc region of human IgG. In this method ILY was used to coat wells in a 96-well plate, the wells were incubated with different concentrations of commercial human Fc fragments, and Fc fragment binding was detected using an anti-human Fc fragment-HRP antibody. This result further supports the conclusion that ILY-binding human IgG binds to ILY by the Fab region.

In order to determine the *in vivo* effect of ILY-binding human IgG, we first treated *hCD59RBC*^{+/-} with an intravenous (IV) injection of different doses of the ILY-binding human IgG. 15 minutes later, we injected these mice with 285 ng ILY/g body weight (3 times lethal dose (LD) of ILY in untreated *hCD59RBC*^{+/-} mice). We found that the survival percentage of *hCD59RBC*^{+/-} pre-treated with 1 and 0.75 µg ILY-binding human IgG/g body weight was 89% (8/9) and 62.5% (5/8), respectively. This result confirms that the ILY-binding human IgG blocks ILY function *in vivo* and suggests that an anti-ILY antibody may be useful for the treatment of SI infectious disease.

### 4. Functional activity of modified recombinant ILY

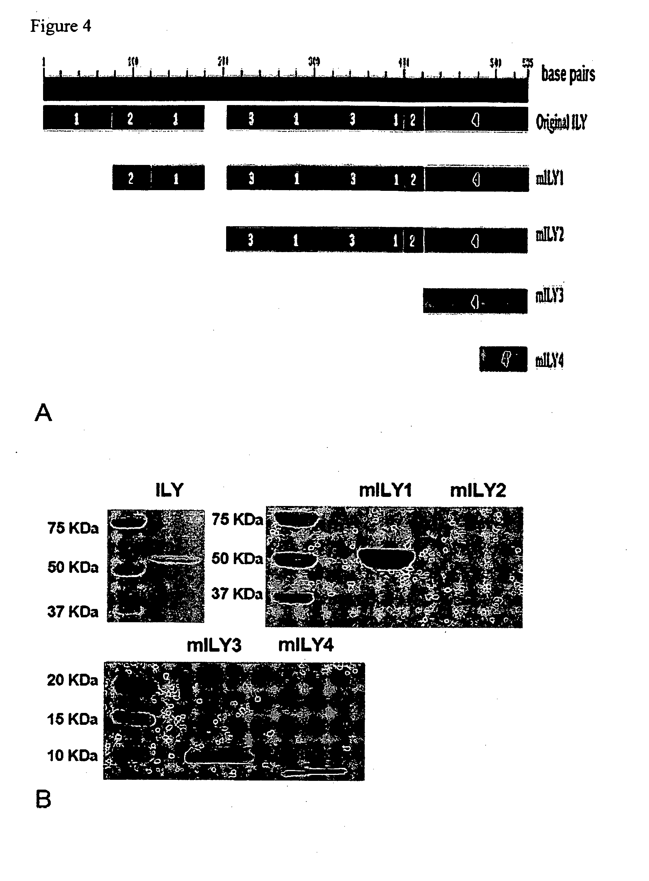

We generated a panel of modified recombinant ILY fragments (mILY1-4). Sequences encoding the fragments were cloned into an expressing vector such that the fragment was fused to a His tag (Fig. 4A). The mILY1 fragment has approximately 80 amino acids deleted at the N terminus of ILY. The mILY2 fragment has approximately 200 amino acids deleted at the N terminus of ILY. These proteins were expressed in an *E. coli* expressing system and further purified by His-column (Fig. 4B).

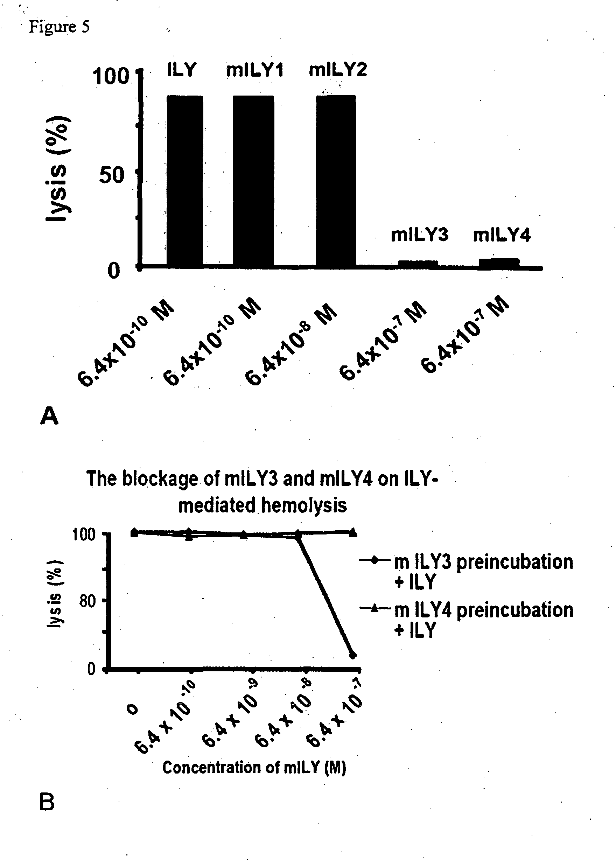

These fragments were tested in the human RBC lysis assay described above. The mDLY1 fragment retains similar lytic activity as full length ILY. The mILY2 fragment retained less than 1% of the lytic activity of full length ILY. The mLY3 and mILY4 fragments did not induce lysis of RBCs even at 10³ fold greater concentrations (Fig. 5A).

In order to test whether either mILY3 or mILY4 can block the lytic effect of full length ILY, human erythrocytes were preincubated with the different concentrations of mILY3 or mILY4 followed by incubation with full length ILY. After preincubation, the mILY3 fragment blocked ILY mediated lysis (Fig. 5B). These data indicates that mILY3, containing the entire domain 4, preserves the functional features for binding to human CD59.

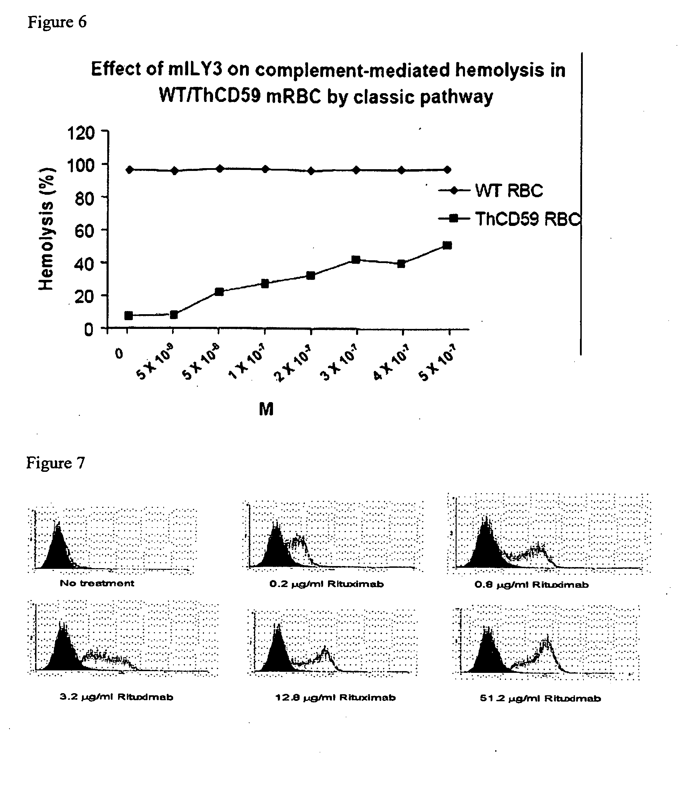

Fig. 6 shows that the preincubation of ILY3 with ThCD59RBC's increases the sensitivity of hCD59RBC transgenic mouse erythrocytes to complement mediated hemolysis. This result confirms that ILY3 block hCD59 function.

### 5. Generation of rituximab-resistance RAMOS cell lines with overexpression of hCD59 in RAMOS membrane

By following the procedure developed in Takei et al. (Leuk Res 30, 625-31 (2006)), we established rituximab-resistant RAMOS cell lines which overexpress human CD59. RAMOS, a B-cell non-Hodgkin lymphoma cell line was repeatedly exposed to complement (human serum) and gradually increasing concentrations of rituximab in vitro. Expression of CD59 on these resistant cells was analyzed by flow cytometry. RAMOS cells that are resistant to gradually increasing concentrations of rituximab (0.2, 0.8, 3.2, 12.8 and 51.2 µg/ml) and 10% of human serum gradually express increased level of human CD59 (Fig. 7).

### 6. mILY3 decreases resistance to rituximab in rituximab-resistant RAMOS cells

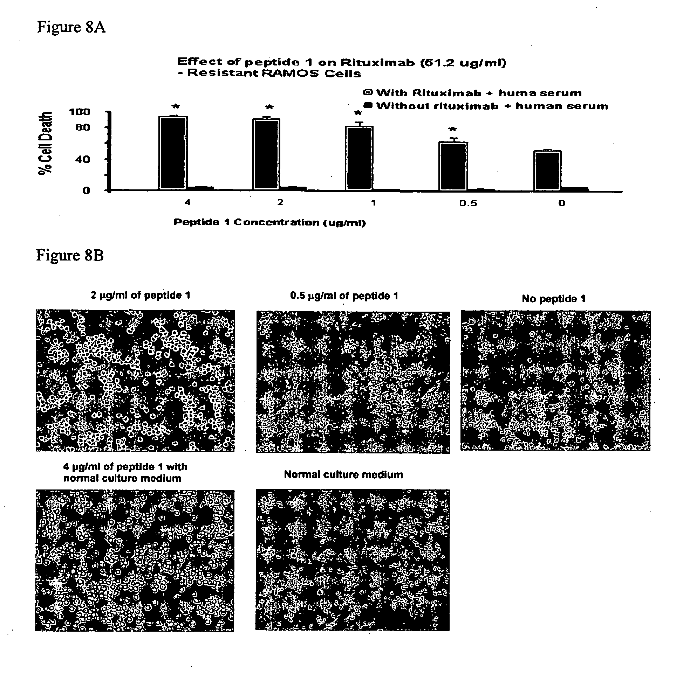

In order to test whether a peptide derived from domain 4 of ILY can inhibit hCD59 function and facilitate rituximab treatment of cancer, we incubated mILY3 with the cells resistant to 51.2 µg/ml rituximab which expressed the highest level of human CD59. RAMOS cells resistant to 51.2 µg/ml rituximab were treated with different concentrations of mILY3 (SEQ ID NO:1), 10 µg/ml rituximab, and 10% of human serum for 1 hour, the cell death was determined using Trypan Blue, a standard method for determining death cells (Fig. 8B).

RAMOS cells treated with varying doses of mILY3 exhibited significantly increased cell death (%) compared to cells not treated with mILY3 (Figs. 8A and 8B). The IL₅₀ of mILY3 was calculated at 33 nM. These data indicate that mILY3 (SEQ ID NO:1), derived from ILY domain 4, decreases rituximab resistance in rituximab-resistant cells in vitro (Figs. 2A and 2B). Significantly, treatment of RAMOS cells with different concentrations of mILY3 alone did not induce cell death (Fig. 8), suggesting that mILY3 alone does not have a toxic effect on these cells.

Further disclosed are the following items 1-14:
1. A substantially pure polypeptide comprising an ILY domain 4 polypeptide.
2. The substantially pure polypeptide of 1, wherein said ILY domain 4 polypeptide comprises a sequence selected from SEQ ID NO: 1 and SEQ ID NO:2, or a fragment thereof, wherein said fragment has ILY domain 4 activity.
3. The substantially pure polypeptide of, 2, wherein the length of said fragment is at least 10, 20, 30, 40, 50, 60, 70, 80, 90, or 100 amino acids.
4. The substantially pure polypeptide of 2, wherein the length of said fragment is fewer than 531, 500, 400, 300, 200, 100, 50, 40, 30, 20, or 10 amino acids.
5. The substantially pure polypeptide of any one of 1-4, wherein said substantially pure polypeptide is a fusion protein.
6. The substantially pure polypeptide of 1, wherein said ILY domain 4 polypeptide comprises a sequence selected from SEQ ID NO: 1 and SEQ ID NO:2.
7. A pharmaceutical composition comprising a substantially pure polypeptide of any one of 1-6 and a therapeutic antibody.
8. The pharmaceutical composition of 7, wherein said therapeutic antibody is selected from the group consisting of rituximab, MT201, 17-1A, herceptin, alemtuzumab, lym-1, bevacizumab, cetuximab, and IL-2 receptor alpha-directed monoclonal antibodies.
9. A method for treating a proliferative disease in patient in need thereof, said method comprising administering to said patient a substantially pure ILY domain 4 polypeptide of any one of 1-6 and a therapeutic antibody, wherein said ILY domain 4 polypeptide and said therapeutic antibody are administered simultaneously, or within 14 days of each other, in amounts that together are sufficient to treat said proliferative-disease.
10. The method of 9, wherein said therapeutic antibody is selected from a group consisting of rituximab, MT201, 17-1A, herceptin, alemtuzumab, lym-1, bevacizumab, cetuximab, and IL-2 receptor alpha-directed monoclonal antibodies.
11. The method of 10, wherein said ILY domain 4 polypeptide and said therapeutic antibody are administered simultaneously.
12. The method of 11, wherein said ILY domain 4 polypeptide is formulated together with said therapeutic antibody.
13. The method of 9, wherein said ILY domain 4 polypeptide comprises a sequence selected from SEQ ID NO:1 and SEQ ID NO:2, or a fragment thereof, wherein said fragment has ILY domain 4 activity
14. The method of 9, wherein said proliferative disease is characterized by neoplastic cells expressing CD59.

## Claims

1. A pharmaceutical composition comprising a substantially pure polypeptide comprising an ILY domain 4 polypeptide and a therapeutic antibody, wherein said substantially pure polypeptide and said therapeutic antibody are separate molecules, and wherein the said substantially pure polypeptide is at least 75 %, by weight, pure.

2. The pharmaceutical composition of claim 1, wherein said ILY domain 4 polypeptide comprises a sequence selected from SEQ ID NO:1 and SEQ ID NO:2.

3. The pharmaceutical composition of any one of claims 1-2, wherein said substantially pure polypeptide is a fusion protein.

4. The pharmaceutical composition of claim 1, wherein said ILY domain 4 polypeptide comprises a sequence selected from SEQ ID NO:1 and SEQ ID NO:2.

5. The pharmaceutical composition of claim 1, wherein said therapeutic antibody is selected from the group consisting of rituximab, MT201, 17-1A, herceptin, alemtuzumab, lym-1, bevacizumab, cetuximab, and IL-2 receptor alpha-directed monoclonal antibodies.

6. The substantially pure ILY domain 4 polypeptide as defined in any one of claims 1-4 and/or a therapeutic antibody for use in a method for treating a proliferative disease in patient having said proliferative disease, said method comprising administering to said patient said polypeptide and said antibody, wherein said ILY domain 4 polypeptide and said therapeutic antibody are administered simultaneously, or within 14 days of each other, in amounts that together are sufficient to treat said proliferative disease, wherein said substantially pure polypeptide and said therapeutic antibody are separate molecules.

7. The polypeptide and/or antibody for use of claim 6, wherein said therapeutic antibody is selected from a group consisting of rituximab, MT201, 17-1A, herceptin, alemtuzumab, lym-1, bevacizumab, cetuximab, and IL-2 receptor alpha-directed monoclonal antibodies.

8. The polypeptide and/or antibody for use of claim 7, wherein said ILY domain 4 polypeptide and said therapeutic antibody are administered simultaneously.

9. The polypeptide and/or antibody for use of claim 8, wherein said ILY domain 4 polypeptide is formulated together with said therapeutic antibody.

10. The polypeptide and/or antibody for use of claim 6, wherein said ILY domain 4 polypeptide comprises a sequence selected from SEQ ID NO:1 and SEQ ID NO:2.

11. The polypeptide and/or antibody for use of claim 6, wherein said proliferative disease is **characterized by** neoplastic cells expressing CD59.

12. The polypeptide and/or antibody for use of any of claims 6 to 8, 10 or 11, wherein said ILY domain 4 polypeptide and said therapeutic antibody are formulated separately.

13. The polypeptide and/or antibody for use according to any of claims 6 to 12, wherein the antibody is an anti-cancer antibody.

14. The substantially pure ILY domain 4 polypeptide as defined in any one of claims 1-4 or 10 for use in a method for sensitizing cancer cells to antibody-based anticancer treatments.

15. The polypeptide for use according to claim 14, with the features as defined in any of claims 5 to 13.

## Patentansprüche

1. Pharmazeutische Zusammensetzung, umfassend ein im Wesentlichen reines Polypeptid, umfassend ein ILY-Domäne-4-Polypeptid, und einen therapeutischen Antikörper, wobei das im Wesentlichen reine Polypeptid und der therapeutische Antikörper getrennte Moleküle sind, und wobei das im Wesentlichen reine Polypeptid mindestens 75 Gew.-% rein ist.

2. Pharmazeutische Zusammensetzung nach Anspruch 1, wobei das ILY-Domäne-4-Polypeptid eine Sequenz umfasst, die ausgewählt ist aus SEQ ID NO: 1 und SEQ ID NO: 2.

3. Pharmazeutische Zusammensetzung nach einem beliebigen der Ansprüche 1-2, wobei das im Wesentlichen reine Polypeptid ein Fusionsprotein ist.

4. Pharmazeutische Zusammensetzung nach Anspruch 1, wobei das ILY-Domäne-4-Polypeptid eine Sequenz umfasst, die ausgewählt ist aus SEQ ID NO: 1 und SEQ ID NO: 2.

5. Pharmazeutische Zusammensetzung nach Anspruch 1, wobei der therapeutische Antikörper ausgewählt ist aus der Gruppe, bestehend aus Rituximab, MT201, 17-1A, Herceptin, Alemtuzumab, Lym-1, Bevacizumab, Cetuximab und auf IL-2-Rezeptor-alpha gerichteten monoklonalen Antikörpern.

6. Im Wesentlichen reines ILY-Domäne-4-Polypeptid, wie in einem beliebigen der Ansprüche 1-4 definiert, und/oder ein therapeutischer Antikörper zur Verwendung in einem Verfahren zum Behandeln einer proliferativen Krankheit in einem Patienten, der die proliferative Krankheit aufweist, wobei das Verfahren Verabreichen des Polypeptids und des Antikörpers an den Patienten umfasst, wobei das ILY-Domäne-4-Polypeptid und der therapeutische Antikörper simultan oder innerhalb von 14 Tagen voneinander in Mengen verabreicht werden, die zusammen ausreichend sind, um die proliferative Krankheit zu behandeln, wobei das im Wesentlichen reine Polypeptid und der therapeutische Antikörper getrennte Moleküle sind.

7. Polypeptid und/oder Antikörper zur Verwendung nach Anspruch 6, wobei der therapeutische Antikörper ausgewählt ist aus einer Gruppe, bestehend aus Rituximab, MT201, 17-1A, Herceptin, Alemtuzumab, Lym-1, Bevacizumab, Cetuximab und auf IL-2-Rezeptor-alpha gerichteten monoklonalen Antikörpern.

8. Polypeptid und/oder Antikörper zur Verwendung nach Anspruch 7, wobei das ILY-Domäne-4-Polypeptid und der therapeutische Antikörper simultan verabreicht werden.

9. Polypeptid und/oder Antikörper zur Verwendung nach Anspruch 8, wobei das ILY-Domäne-4-Polypeptid zusammen mit dem therapeutischen Antikörper formuliert wird/ist.

10. Polypeptid und/oder Antikörper zur Verwendung nach Anspruch 6, wobei das ILY-Domäne-4-Polypeptid eine Sequenz umfasst, die ausgewählt ist aus SEQ ID NO: 1 und SEQ ID NO: 2.

11. Polypeptid und/oder Antikörper zur Verwendung nach Anspruch 6, wobei die proliferative Krankheit **gekennzeichnet ist durch** neoplastische Zellen, die CD59 exprimieren.

12. Polypeptid und/oder Antikörper zur Verwendung nach einem beliebigen der Ansprüche 6 bis 8, 10 oder 11, wobei das ILY-Domäne-4-Polypeptid und der therapeutische Antikörper separat formuliert sind/werden.

13. Polypeptid und/oder Antikörper zur Verwendung nach einem beliebigen der Ansprüche 6 bis 12, wobei der Antikörper ein Anti-Krebs-Antikörper ist.

14. Im Wesentlichen reines ILY-Domäne-4-Polypeptid, wie in einem beliebigen der Ansprüche 1-4 oder 10 definiert, zur Verwendung in einem Verfahren zum Sensibilisieren von Krebszellen gegen Antikörper-basierte Anti-Krebs-Behandlungen.

15. Polypeptid zur Verwendung gemäß Anspruch 14, mit den Merkmalen wie in (einem) beliebigen der Ansprüche 5 bis 13 definiert.

## Revendications

1. Composition pharmaceutique comprenant un polypeptide sensiblement pur comprenant un polypeptide du domaine 4 de ILY et un anticorps thérapeutique, dans laquelle ledit polypeptide sensiblement pur et ledit anticorps thérapeutique sont des molécules séparées, et dans laquelle ledit polypeptide sensiblement pur est au moins pur à 75 % en poids.

2. Composition pharmaceutique selon la revendication 1, dans laquelle ledit polypeptide du domaine 4 de ILY comprend une séquence choisie parmi SEQ ID N° 1 et SEQ ID N° 2.

3. Composition pharmaceutique selon l'une quelconque des revendications 1 à 2, dans laquelle ledit polypeptide sensiblement pur est une protéine de fusion.

4. Composition pharmaceutique selon la revendication 1, dans laquelle ledit polypeptide du domaine 4 de ILY comprend une séquence choisie parmi SEQ ID N° 1 et SEQ ID N° 2.

5. Composition pharmaceutique selon la revendication 1, dans laquelle ledit anticorps thérapeutique est choisi dans le groupe constitué par le rituximab, MT201, 17-1A, l'herceptine, l'alemtuzumab, le lym-1, le bévacizumab, le cétuximab et les anticorps monoclonaux dirigés contre le récepteur alpha de l'IL-2.

6. Polypeptide du domaine 4 de ILY sensiblement pur tel que défini selon l'une quelconque des revendications 1 à 4 et/ou un anticorps thérapeutique pour une utilisation dans une méthode de traitement d'une maladie proliférative chez un patient présentant ladite maladie proliférative, ladite méthode comprenant l'administration audit patient dudit polypeptide et dudit anticorps, dans lequel ledit polypeptide du domaine 4 de ILY et ledit anticorps thérapeutique sont administrés simultanément, ou dans les 14 jours l'un de l'autre, en des quantités qui, ensemble, sont suffisantes pour traiter ladite maladie proliférative, dans lequel ledit polypeptide sensiblement pur et ledit anticorps thérapeutique sont des molécules séparées.

7. Polypeptide et/ou anticorps pour une utilisation selon la revendication 6, dans lequel ledit anticorps thérapeutique est choisi dans le groupe constitué par le rituximab, MT201, 17-1A, l'herceptine, l'alemtuzumab, le lym-1, le bévacizumab, le cétuximab et les anticorps monoclonaux dirigés contre le récepteur alpha de l'IL-2.

8. Polypeptide et/ou anticorps pour une utilisation selon la revendication 7, dans lequel ledit polypeptide du domaine 4 de ILY et ledit anticorps thérapeutique sont administrés simultanément.

9. Polypeptide et/ou anticorps pour une utilisation selon la revendication 8, dans lequel ledit polypeptide du domaine 4 de ILY est formulé avec ledit anticorps thérapeutique.

10. Polypeptide et/ou anticorps pour une utilisation selon la revendication 6, dans lequel ledit polypeptide du domaine 4 de ILY comprend une séquence choisie parmi SEQ ID N° 1 et SEQ ID N° 2.

11. Polypeptide et/ou anticorps pour une utilisation selon la revendication 6, dans lequel ladite maladie proliférative est **caractérisée par** des cellules néoplasiques exprimant CD59.

12. Polypeptide et/ou anticorps pour une utilisation selon l'une quelconque des revendications 6 à 8, 10 ou 11, dans lequel ledit polypeptide du domaine 4 de ILY et ledit anticorps thérapeutiques sont formulés séparément.

13. Polypeptide et/ou anticorps pour une utilisation selon l'une quelconque des revendications 6 à 12, dans lequel l'anticorps est un anticorps anticancéreux.

14. Polypeptide du domaine 4 de ILY sensiblement pur tel que défini dans l'une quelconque des revendications 1 à 4 ou 10 pour une utilisation dans une méthode destinée à la sensibilisation de cellules cancéreuse vis-à-vis de traitements anticancéreux à base d'anticorps.

15. Polypeptide pour une utilisation selon la revendication 14, avec les caractéristiques telles que définies dans l'une quelconque des revendications 5 à 13.
